# FASCICULE DE BREVET EUROPEEN

(11) **EP 0 669 995 B1**
(45) Date de publication et mention de la délivrance du brevet: **22.01.1997**
(21) Numéro de dépôt: 94900205.9
(22) Date de dépôt: 18.11.1993
(51) Int. Cl.: D04H 13/00, A61F 13/46

(54) **MATERIAU NON-TISSE MULTICOUCHE ET ARTICLE D'HYGIENE ABSORBANT COMPORTANT UN TEL MATERIAU**
MEHRSCHICHTIGES VLIESSTOFFMATERIAL UND SAUGFÄHIGES, HYGIENISCHES PRODUKT ZUSAMMENGESETZT MIT EINEM SOLCHEN VERBAND
NON WOVEN MULTILAYER MATERIAL AND HYGIENIC ABSORBENT ARTICLE COMPRISING SUCH MATERIAL

(30) Priorité: 20.11.1992 FR 9213997
(43) Date de publication de la demande: 06.09.1995
(73) Titulaire: PEAUDOUCE, 59126 Linselles (FR)
(72) Inventeur: KOCZAB, Jean-Pierre, F-59910 Bondues (FR)
(74) Mandataire: Casalonga, Axel
(86) Numéro de dépôt international: FR9301131
(87) Numéro de publication internationale: WO9412713

(56) Documents cités:
- EP-A- 0 232 729
- EP-A- 0 252 041
- EP-A- 0 306 262
- EP-A- 0 352 208
- WO-A-91/11164
- DE-A- 3 029 315
- US-A- 3 206 351
- US-A- 4 223 677

## Description

L'invention concerne, d'une manière générale, un matériau non-tissé composite composé de plusieurs couches ou nappes de fibres textiles.

Plus particulièrement l'invention concerne un matériau non-tissé composite composé de plusieurs couches dans lesquelles les fibres ont un denier (mesure du diamètre ou de la grosseur des fibres) décroissant, dans un sens donné, d'une couche successive à l'autre.

L'invention concerne également un article d'hygiène absorbant tel qu'une couche-culotte ou garniture pour incontinent comprenant comme voile ou feuille de surface (également désigné par voile ou feuille de couverture) un voile constitué du matériau non-tissé multicouche dont la couche formée avec les fibres de plus faible denier se trouve directement sur le coussin absorbant de l'article.

L'invention concerne aussi un article d'hygiène absorbant tel qu'une couche-culotte ou garniture comprenant, en plus du voile de surface, une bande de zone d'entre-jambes, constituée du matériau non-tissé multicouche, disposée sur le voile de surface, ou entre le voile de surface et le coussin absorbant de l'article, dont la couche de fibres formée des fibres de plus faible dernier se trouve directement en contact avec, respectivement, le voile de surface ou le coussin absorbant.

Les articles d'hygiène absorbant, tels que des couches-culottes et garnitures pour incontinent, comportent en général un coussin ou matelas de matériau absorbant sur lequel est disposé un voile ou feuille de surface. Ce voile ou feuille de surface lors de l'utilisation des articles se trouve en contact direct avec la peau et a pour objet d'isoler la peau du coussin absorbant humide. Ce voile doit avoir certaines propriétés, à savoir un contact agréable avec la peau, permettre une traversée rapide des fluides corporels jusqu'au coussin absorbant, assurer une bonne diffusion des liquides sur toute la surface du coussin absorbant, et éviter une remontée des fluides corporels absorbés par le coussin vers la surface supérieure du voile.

On a récemment développé des coussins ou matelas absorbants qui contiennent des particules de matériau superabsorbant, généralement disposées entre deux couches de fibres, elles-mêmes comprises entre une couche de base généralement en matériau non-tissé et une couche supérieure en contact avec la peau généralement en ouate de cellulose ou en un matériau non-tissé. Un tel type de coussin est décrit dans EP-A-0232729. Afin d'obtenir une efficacité maximum des matériaux superabsorbants contenus dans ces coussins, il est souhaitable que le liquide soit diffusé ou réparti sur toute la surface supérieure du matelas absorbant. Les voiles ou feuilles de surface disposés à la surface supérieure de tels coussins sont généralement constitués d'une ou plusieurs couches d'un matériau non-tissé.

Le document FR-A-2 588 285 décrit un textile non-tissé multicouche ayant au moins deux couches de voile non-tissé, l'une des couches étant formée de fibres de section transversale bilobée et l'autre couche étant formée de fibres de section transversale trilobée. Chaque couche de voile est de préférence obtenue par la technique de liaison au filage (non-tissé "spun") et les deux couches de voile sont réunies pour former le non-tissé multicouche par liaison thermique en des zones compactées discontinues.

Le document WO 87/07117 décrit un article d'hygiène absorbant comprenant un corps absorbant entouré d'une enveloppe. Cette enveloppe ou voile de surface est constituée de deux couches de matériau non-tissé. La première couche de matériau non-tissé, en contact avec la peau de l'utilisateur, est constituée d'une mince couche de tissu fibreux lié au filage (non-tissé "spun") en un matériau hydrophobique, et la seconde couche en contact avec le corps absorbant est une couche fibreuse hydrophobique de tissu de fibres lié par fusion, de construction similaire à la première couche. Ces deux couches de voile de surface ne sont pas liées entre elles dans la zone destinée à venir en contact avec le corps de l'utilisateur.

Le document WO 88/05269 concerne un voile de surface pour un article absorbant jetable composé d'au moins deux couches de non-tissé qui peuvent être identiques ou différentes et qui sont réunies par des lignes d'adhésif formant un motif ouvert.

Bien que de tels voiles ou feuilles de surface présentent une vitesse de pénétration ou de traversée des fluides corporels convenables et assurent une certaine diffusion du liquide sur la surface du matelas ou coussin absorbant, il est toujours souhaitable d'améliorer ces caractéristiques. De plus, il est également souhaitable d'obtenir une réduction maximale de toute remontée des fluides corporels afin d'obtenir un voile de surface qui s'assèche rapidement et qui ne se remouille pas.

La présente invention a donc pour but de fournir un matériau non-tissé qui ait une vitesse de pénétration ou temps de transpercement par les fluides corporels amélioré, s'assèche rapidement, assure une bonne diffusion des fluides corporels sur toute la surface de matelas absorbant, et réduise au maximum toute remontée des fluides corporels absorbés par le matelas.

L'invention a également pour but de fournir un article d'hygiène absorbant tel qu'une couche-culotte ou garniture pour incontinent, comportant un coussin ou matelas absorbant pourvu à sa surface d'un voile ou feuille de surface fait du matériau non-tissé multicouche de la présente invention.

L'invention a également pour but de fournir un article d'hygiène absorbant tel qu'une couche-culotte ou garniture pour incontinent comportant un coussin ou matelas absorbant, un voile de surface en matériau non-tissé classique, et en complément du voile de surface classique une bande de largeur analogue à celle de la zone d'entre-jambes du coussin absorbant et de longueur analogue à celle de ce coussin, constituée d'un matériau non-tissé multicouche selon l'invention.

Selon la présente invention on réalise un matériau non-tissé multicouche qui comprend au moins deux couches superposées de fibres textiles naturelles ou synthétiques, réunies entre elles, caractérisé par le fait que les fibres des couches successives ont un denier décroissant progressivement, dans un sens donné, d'une couche successive à l'autre.

L'invention fournit également un article d'hygiène absorbant qui comprend une couche externe en matériau imperméable aux fluides corporels, un coussin absorbant perméable aux fluides corporels et lié à la couche externe, et un voile de surface perméable aux fluides corporels et lié à la couche externe caractérisé en ce que le voile de surface est constitué par un matériau non-tissé multicouche comprenant au moins deux couches superposées de fibres textiles naturelles ou synthétiques réunies entre elles, les fibres des couches successives ayant un denier décroissant progressivement d'une couche successive à l'autre, la couche ayant les fibres de plus faible denier étant située directement sur la surface interne du coussin absorbant.

Selon une autre réalisation de l'invention, on fournit un article d'hygiène absorbant qui comprend une couche externe en matériau impermable aux fluides corporels, un coussin absorbant perméable aux fluides corporels et lié à la couche externe, un voile de surface en matériau perméable aux fluides corporels et lié à la couche externe, et en complément à ce voile de surface une bande supplémentaire de zone d'entre-jambes de largeur semblable à celle de la zone d'entre-jambes du coussin absorbant et de longueur analogue à celle du coussin et constituée d'au moins deux couches superposées de fibres textiles naturelles ou synthétiques réunies entre elles, les fibres des couches successives ayant un denier décroissant progressivement d'une couche successive à l'autre, la couche de fibres de plus faible denier constituant, dans l'article d'hygiène, la couche la plus externe de la bande, c'est-à-dire la couche la plus proche du coussin absorbant, et la couche de fibres de plus grand dernier constituant la couche la plus interne de la bande, c'est-à-dire la couche la plus éloignée du coussin absorbant.

Une caractéristique importante de ce matériau non-tissé réside dans le fait que les différentes couches superposées sont constituées de fibres dont le denier (unité mesurant la grosseur ou le diamètre des fibres) varie d'une couche à la suivante selon une décroissance progressive depuis une couche interne ou de surface jusqu'à une couche externe ou de base. On obtient ainsi une structure fibreuse dont le diamètre des pores diminue de la couche interne à la couche externe. On peut utiliser pour la fabrication des couches en non-tissé toutes fibres naturelles ou synthétiques convenables, par exemple des fibres de cellulose, viscose, polyester, polyéthylène, polypropylène ou copolymères d'éthylène-propylène. Les différentes couches peuvent être composées de fibres de même nature ou de nature différente. Ces couches ou nappes de fibres textiles peuvent être formées par tout procédé utilisé pour la fabrication de non-tissé tel que par liaison au filage (spun bonded), laçage au filage (spun lace), liaison chimique, liaison thermique, aiguilletage, nappage par flux d'air (air-laid) ou enchevêtrement par jets d'eau. Comme indiqué, le matériau non-tissé multicouche comporte au moins deux couches de fibres de denier différent, mais peut comporter un nombre supérieur de couches, par exemple trois ou plus. Dans une réalisation, recommandée, le matériau non-tissé selon l'invention se compose de deux couches de fibres de denier décroissant de la couche interne à la couche externe. Dans cette réalisation, la couche de fibres de plus grand dernier a, en général, un denier compris entre 3,3 et 6,6 cependant que la couche de plus faible denier a, en général, un denier compris entre 1,5 et 3,3. Dans une autre réalisation de l'invention, le matériau non-tissé se compose de cinq couches de fibres de dernier décroissant de la couche interne à la couche externe. Dans cette réalisation comprenant cinq couches, le denier des fibres est de préférence choisi, pour chacune des couches successives, de la couche de fibres de plus grand denier à la couche de fibres de plus faible denier, dans les gammes suivantes :
. couche 1 (couche la plus interne) : 6,6 à 9
. couche 2 : 4,4 à 6,5
. couche 3 : 3,3 à 4,3
. couche 4 : 1,7 à 3,2
. couche 5 (couche la plus externe) : 0,8 à 1,6.

En général, les couches du non-tissé multicouche selon l'invention ont un grammage compris entre 5 et 50 g/m², de préférence entre 5 et 30 g/m². De plus, dans une réalisation recommandée, on peut traiter certaines des couches avec des agents de surface ou d'ensimage appropriés bien connus dans la technique pour leur conférer des caractéristiques d'hydrophilie. Ces couches traitées ont pour avantage d'accroître la vitesse de traversée par les fluides corporels, cependant que les couches non traitées ont des propriétés de diffusion des fluides dans leur plan ce qui assure une meilleure répartition ou diffusion des fluides corporels sur toute la surface du matelas absorbant. De manière avantageuse, on peut alterner les couches à caractère hydrophile et hydrophobe. Le caractère hydrophile ou hydrophobe des différentes couches peut être obtenu en utilisant des procédés de fabrication différents classiques pour chacune des couches.

Les différentes couches du matériau non-tissé multicouche sont réunies entre elles par toute technique appropriée, par exemple liaison thermique ou liage mécanique, en particulier par aiguilletage ou par enchevêtrement par jets d'eau. Un mode recommandé de liaison des couches selon l'invention est l'aiguilletage.

Un tel matériau est particulièrement utile pour la fabrication de voiles ou feuilles de surface ou de bandes de zone d'entre-jambes utilisées en remplacement ou en complément du voile de surface classique dans des articles d'hygiène absorbants tels que des couches-culottes et garnitures pour incontinent.

Lorsqu'il est utilisé comme voile de surface d'un article d'hygiène absorbant, en remplacement du voile de surface classique, le matériau non-tissé multicouche de la présente invention est disposé directement sur le matelas absorbant de l'article avec la couche constituée de fibres de plus faible denier en contact avec la surface interne du coussin. Par conséquent, la couche de fibres de plus grand denier se trouve être la couche supérieure ou interne du voile qui se trouvera en contact avec la peau de l'utilisateur. De ce fait, les couches ayant un diamètre de pores plus grand se trouvent à la partie supérieure ou interne du voile et les couches ayant un diamètre de pores plus faible à la partie inférieure ou externe du voile. Ainsi, on obtient une traversée rapide des couches supérieures du voile ce qui assure un assèchement rapide de ces couches supérieures et un confort accru de l'utilisateur, cependant que la présence de pores de plus petits diamètres dans les couches inférieures du voile, et en particulier directement au-dessus du coussin réduit au maximum toute remontée des fluides corporels qui ont été absorbés par le coussin absorbant.

Lorsqu'il est utilisé en complément du voile de surface classique d'un article d'hygiène absorbant, le matériau non-tissé multicouche selon l'invention constitue, de préférence, une bande de largeur analogue à celle du coussin absorbant dans la zone d'entre-jambes. La longueur de la bande peut correspondre à celle de l'article d'hygiène, mais sera de préférence égale à celle du coussin absorbant. La bande en matériau non-tissé multicouche selon l'invention peut être disposée, dans la zone d'entre-jambes, soit sur le voile en non-tissé classique soit entre le voile en non-tissé classique et le coussin absorbant. Dans les deux cas la couche la plus interne de la bande, c'est-à-dire la couche la plus éloignée du coussin absorbant, est la couche de fibres de plus grand denier, et par conséquent la couche la plus externe de la bande, c'est-à-dire la couche la plus proche du coussin absorbant, est la couche de fibres de plus faible dernier. Lorsque cette bande supplémentaire est située au-dessus du voile de surface classique elle peut être liée à la surface interne du voile de surface par tout procédé classique tel que par exemple collage, thermoscellage, scellage aux ultrasons ou aiguilletage. Lorsque cette bande supplémentaire en matériau non-tissé multicouche selon l'invention se trouve située entre le voile de surface et le coussin absorbant, elle peut être liée soit à la surface externe du voile de surface soit à la surface interne du coussin absorbant par tout procédé classique tel que par exemple collage, thermoscellage, scellage aux ultrasons ou aiguilletage.

La suite de la description se réfère aux figures annexées qui représentent, respectivement :
figure 1, une réalisation recommandée du matériau non-tissé composite multicouche selon l'invention;
figure 2, une vue de dessus, en partie arrachée, d'un article d'hygiène absorbant, tel qu'une couche-culotte, comportant un voile de surface en matériau selon la figure 1;
figure 3, une vue en coupe faite selon la ligne III-III de la figure 2;
figure 4, une autre réalisation d'un matériau non-tissé composite multicouche selon l'invention;
figure 5 une vue de dessus, en partie arrachée, d'un article d'hygiène absorbant, tel qu'une couche-culotte, comportant un voile de surface en matériau selon la figure 4;
figure 6, une vue en coupe faite selon la ligne VI-VI de la figure 5;
figure 7, une vue de dessus; en partie arrachée, d'un article d'hygiène absorbant, tel qu'une couche-culotte comportant une bande de zone d'entrejambes, selon l'invention, disposée sur la surface interne du voile de surface;
figure 8, une vue en coupe faite selon la ligne VIII-VIII de la figure 7;
figure 9, une vue de dessus, en partie arrachée, d'un article d'hygiène absorbant, tel qu'une couche-culotte comportant une bande de zone d'entre-jambes, selon l'invention, disposée entre le voile de surface et le coussin absorbant; et
figure 10, une vue en coupe faite selon la ligne X-X de la figure 9.

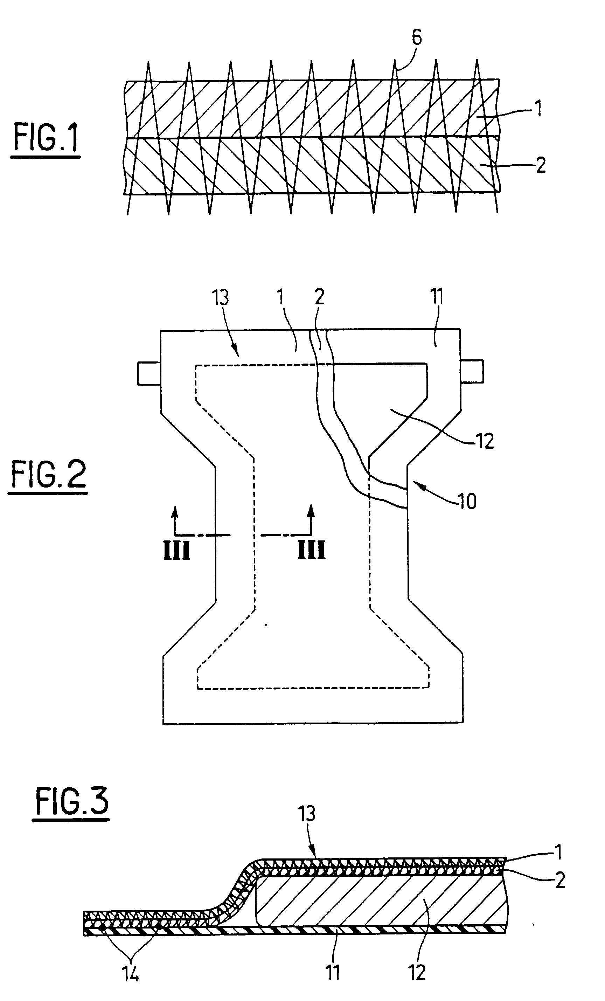

En se référant à la figure 1, on a représenté une première réalisation d'un matériau composite multicouche selon l'invention qui comporte deux couches superposées de fibres de polyester (1, 2) dont les caractéristiques sont les suivantes en partant de la couche supérieure ou interne 1 à la couche inférieure ou externe 2. La couche interne est constituée de fibres de polyester d'un denier compris entre 3,3 et 6,6, de préférence de 6,6, et a un grammage compris entre 5 et 50 g/m², par exemple 41 g/m². La couche externe 2 est constituée de fibres de polyester d'un denier compris entre 1,5 et 3,3, de préférence 3,3 ou 1,5 deniers, et a un grammage compris entre 5 et 50 g/m², par exemple 22g/m². Les couches sont réunies par aiguilletage ou enchevêtrement par jets d'eau. De préférence les couches sont réunies par un aiguilletage 6.

En se référant aux figures 2 et 3, on a représenté un article d'hygiène absorbant 10, tel qu'une couche-culotte, comportant un voile de surface 13 fait en matériau non-tissé composite multicouche selon l'invention. l'article d'hygiène 10 comprend une couche externe 11 en un matériau souple imperméable aux fluides corporels sur laquelle est disposé un matelas ou coussin absorbant 12 perméable aux fluides corporels, de dimension inférieure à la couche externe 11 et lié à la couche externe 11 par tout moyen approprié, par exemple par collage. Sur ce coussin absorbant 12 se trouve un voile de surface 13 perméable aux fluides corporels et de dimension supérieure à celle du matelas absorbant 12 et qui est lié à la couche externe 11 par tout moyen approprié tel que des collages 14. Comme cela est bien connu, la couche externe 11, le coussin absorbant 12 et le voile de surface 13 ont en général la forme d'un sablier comprenant deux parties terminales opposées larges réunies par une partie centrale de zone d'entre-jambes de forme générale rectangulaire et de plus faible largeur. Comme on le voit mieux à la figure 3, dans la présente réalisation le voile de surface 13 est constitué du matériau non-tissé composite à deux couches de la figure 1. La couche inférieure ou externe 2 constituée des fibres de plus faible denier se trouve disposée directement sur le coussin 12 cependant que la couche supérieure ou interne en fibres de plus grand denier constitue la couche qui sera en contact avec la peau de l'utilisateur. Ce voile de surface 13 peut être réuni au coussin absorbant 12 par tout moyen classique tel que collage, thermoscellage, scellage aux ultrasons ou aiguilletage.

On obtient ainsi une couche-culotte ayant des caractéristiques de vitesse de transpercement et de résistance au remouillage par les fluides corporels améliorées.

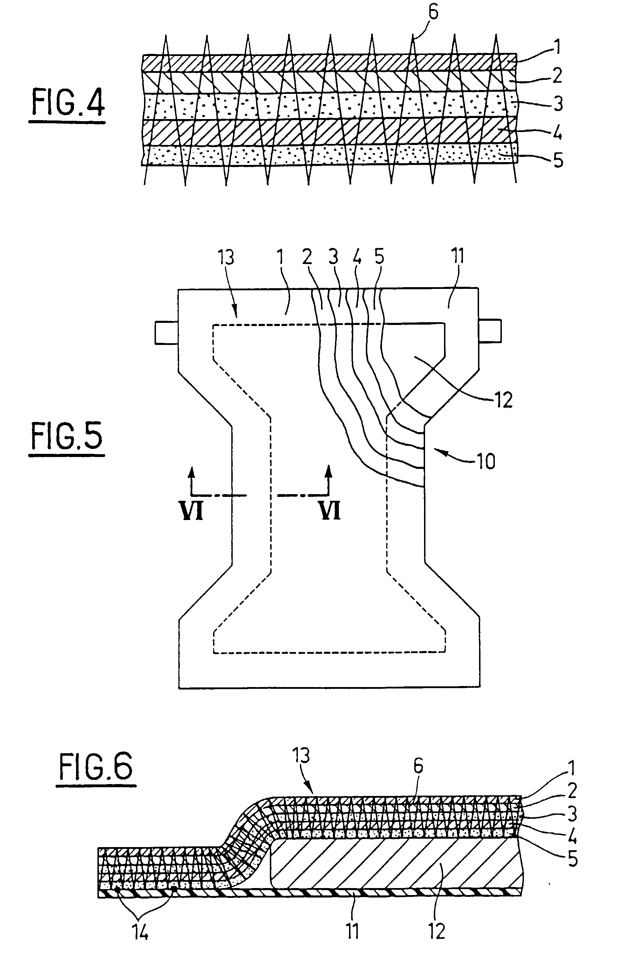

En se référant à la figure 4, on y a représenté une réalisation d'un matériau non-tissé multicouche selon la présente invention particulièrement approprié pour le remplacement du voile de surface classique et qui comporte cinq couches superposées de fibres de polyester (1 à 5) dont les caractéristiques sont les suivantes en partant de la couche supérieure ou interne jusqu'à la couche de fond ou externe :

| | | |
|---|---|---|
| - couche 1 | 6,6 deniers | 30 g/m² |
| - couche 2 | 4,4 deniers | 20 g/m² |
| - couche 3 | 3,3 deniers | 20 g/m² |
| - couche 4 | 1,7 denier | 15 g/m² |
| - couche 5 | 0,8 denier | 15 g/m² |

Les couches sont réunies par un aiguilletage 6 pour obtenir un voile de non-tissé final ayant un grammage de 100 g/m². La densité d'aiguilletage était de 20 coups/cm² et par face. Bien évidemment, comme on l'a indiqué précédemment, une ou plusieurs des couches peuvent être constituées de fibres d'un autre type, tel que par exemple des fibres de viscose, ou de tout autre fibres textiles naturelles ou synthétiques. En particulier l'utilisation de fibres de viscose dans certaines des couches apporte de la rétention à la nappe. De plus, on peut régler l'épaisseur de chaque couche individuelle et également celle du produit final en réglant la densité de l'aiguilletage, lorsque l'on utilise pour la formation de chacune des couches et/ou pour réunir les couches entre elles un procédé d'aiguilletage. Un tel matériau non-tissé multicouche utilisé comme voile de surface ou comme bande de zone d'entre-jambes en complément du voile de surface dans un article d'hygiène absorbant se caractérise par un assèchement rapide du matériau, une vitesse de pénétration ou temps de transpercement par les fluides corporels améliorée, et une bonne diffusion de ces fluides sur toute la surface du matelas absorbant.

En se référant aux figures 5 et 6, on a représenté un article d'hygiène absorbant 10, tel qu'une couche-culotte, comportant un voile de surface 13 fait en matériau non-tissé multicouche selon l'invention. L'article d'hygiène 10 comprend une couche externe 11 en un matériau souple imperméable aux fluides corporels sur laquelle est disposé un matelas ou coussin absorbant 12 perméable aux fluides corporels et de dimension inférieure à la couche externe 11 et lié à la couche externe 11 par tout moyen approprié. Sur ce coussin absorbant 12 se trouve un voile de surface 13 perméable aux fluides corporels et de dimension supérieure à celle du matelas absorbant 12. Comme cela est bien connu, la couche externe 11, le coussin absorbant 12 et le voile de surface 13 ont en général la forme d'un sablier comprenant deux parties terminales opposées larges réunies par une partie centrale de zone d'entre-jambes de forme générale rectangulaire et de plus faible largeur. Comme on le voit mieux sur la figure 6, dans une réalisation recommandée, le voile de surface est constitué de cinq couches 1 à 5 de matériau non-tissé. Le denier des fibres constituant chacune des couches de non-tissé diminue progressivement de la couche supérieure ou interne 1 à la couche de base ou externe 5 qui se trouve directement en contact avec la surface supérieure ou interne du coussin absorbant. L'ensemble des couches 1 à 5 est lié par aiguilletage 6. Le voile de surface 13 en non-tissé multicouche est lié sur son pourtour à la couche externe imperméable 11 par tout moyen convenable, par exemple par un collage 14.

Ainsi la couche 5 du voile de surface 13 constituée des fibres de plus faible denier se trouve directement sur le coussin absorbant 12, cependant que la couche 1 constituée des fibres de plus grand denier se trouve être la couche supérieure ou interne du voile qui sera en contact avec la peau de l'utilisateur. Le voile de surface 13 présente donc un diamètre de pore qui décroît progressivement de la couche supérieure ou interne 1 à la couche de base ou externe 5 ce qui résulte en un assèchement rapide de la surface du voile 13 et un effet de barrière accru vis-à-vis du remouillage par les fluides absorbés par le coussin 12.

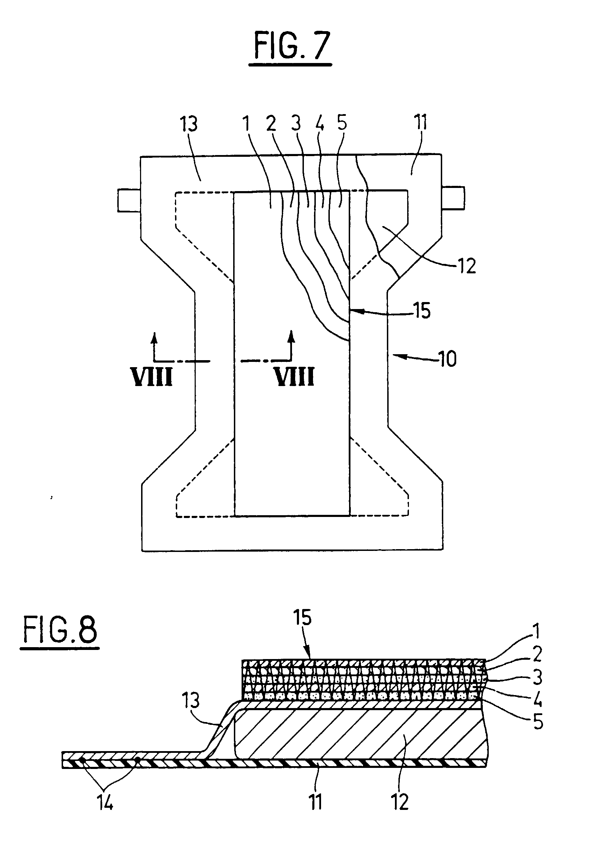

En se référant aux figures 7 et 8, on a représenté un article d'hygiène absorbant 10, tel qu'une couche-culotte semblable à celle des figures 5 et 6 qui comprend une couche externe 11 en un matériau souple imperméable aux fluides corporels sur laquelle est disposé un matelas ou coussin absorbant 12 perméable aux fluides corporels et de dimension inférieure à la couche externe 11. Sur ce coussin absorbant 11 se trouve un voile de surface 13 formé d'un non-tissé classique, par exemple en fibres de polypropylène de 2,2 denier, de dimension analogue à celle de la couche externe 11 et lié à la couche externe par tout moyen approprié, par exemple des collages 14. Sur ce voile de surface 13 est disposé une bande de zone d'entre-jambes 15 perméable aux fluides corporels faite du matériau non-tissé selon l'invention. Cette bande de zone d'entre-jambes 15 est de manière générale de forme rectangulaire ayant une largeur analogue à celle du coussin 12 dans la zone d'entre-jambes et une longueur analogue à celle de ce coussin 12. Selon l'invention, cette bande de zone d'entre-jambes 15 comporte au moins deux couches de non-tissé, dans la réalisation représentée cinq couches 1-5, dont le denier décroît de la couche 1 la plus interne à la couche 5 la plus externe qui se trouve en contact avec le voile de surface 13. Cette bande de zone d'entre-jambes 15 peut être liée à la surface interne du voile de surface en non-tissé classique 13 par tout moyen classique, tel que collage, thermoscellage, scellage aux ultrasons ou aiguilletage.

Comme précédemment la couche 5 en fibres de plus faible denier est la couche de la bande de zone d'entre-jambes qui est la plus proche du coussin absorbant cependant que la couche 1 en fibres de plus grand denier se trouve être la couche la plus éloignée du coussin 12 et, dans cette réalisation, la couche destinée à venir en contact avec le corps de l'utilisateur. Comme précédemment on obtient un assèchement rapide de la ou des couches supérieures de la bande de zone d'entre-jambes et un effet de barrière accru vis-à-vis du remouillage.

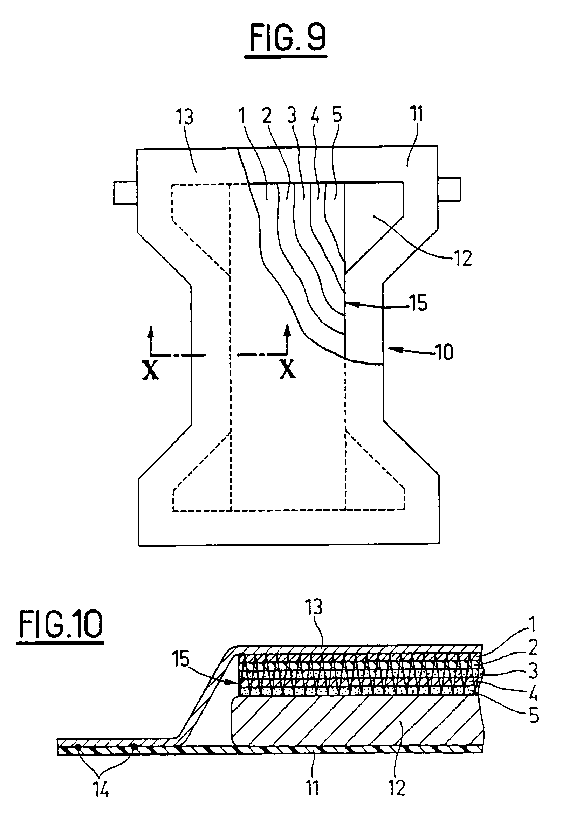

En se référant aux figures 9 et 10 on a représenté un article d'hygiène absorbant 10 analogue à celui des figures 7 et 8 mais dans lequel la bande de zone d'entre-jambes 15 est disposée entre le voile de surface en non-tissé classique 13 et le coussin absorbant 12. Dans cette réalisation, excepté l'emplacement de la bande de zone d'entre-jambes 15, tous les autres éléments sont identiques à ceux des figures 7 et 8. Dans cette réalisation, la bande de zone d'entre-jambes 15 est disposée entre le voile de surface 13 et le coussin 12 de telle sorte que la couche 1 en fibres de plus grand denier soit la couche la plus interne, c'est-à-dire la couche la plus proche du voile de surface 13, et la couche 5 en fibres de plus faible denier soit la couche la plus externe de la bande, c'est-à-dire la couche en contact direct avec la surface interne du coussin absorbant 12. La bande de zone d'entre-jambes 15 en matériau non-tissé multicouche selon l'invention peut être réunie soit à la surface externe du voile de surface 13 soit à la surface interne du coussin absorbant par tout moyen approprié tel que, par exemple collage, thermoscellage, scellage aux ultrasons ou aiguilletage.

La couche-culotte ainsi réalisée présente un assèchement rapide des surfaces internes dans la zone de l'entre-jambes et un effet de barrière accru vis-à-vis du remouillage.

Les exemples suivants sont donnés à titre d'illustration de la présente invention.

### Exemple comparatif A

On a déterminé la vitesse de transpercement et la résistance au remouillage d'une couche-culotte du commerce (Peaudouce Action girl® correspondant à la taille 8-18kg, dont le poids total est de 59,2 g, le poids du coussin absorbant de 48,6 g dont 4,8g de matériau superabsorbant) et qui comporte un voile de surface classique en non-tissé de fibres de polypropylène de type lié au filage (non-tissé "spun") ayant un grammage de 20 g/m². Les résultats sont donnés dans le tableau 1 ci-dessous.

### Exemple comparatif B

On a déterminé la vitesse de transpercement et la résistance au remouillage d'une autre couche-culotte du commerce de taille identique à celle de l'exemple comparatif A et ayant un voile de surface identique (Peaudouce Action boy® dont le poids total est de 59,4 g, le poids du coussin absorbant est de 49 g, dont 4,94 g de matériau superabsorbant). Les résultats sont donnés dans le tableau 1 ci-dessous.

### Exemple comparatif C

On a remplacé le voile de surface de la couche-culotte de l'exemple comparatif B par trois couches superposées de fibres de polyester de 6,6 deniers et d'un grammage de 41g/m², soit un grammage total de 123 g/m². On a déterminé la vitesse de transpercement et la résistance au remouillage. Les résultats sont donnés dans le tableau 1.

### Exemple comparatif D

On a remplacé le voile de surface de la couche-culotte de l'exemple comparatif B par six couches superposées de fibres de polyester de 3,3 deniers et d'un grammage de 22 g/m², soit un grammage total de 132 g/m². On a déterminé la vitesse de transpercement et la résistance au remouillage. Les résultats sont donnés dans le tableau 1.

### Exemple comparatif E

On a remplacé le voile de surface de la couche-culotte de l'exemple comparatif B par six couches de fibres de polyester de 1,5 deniers et d'un grammage de 22g/m², soit un grammage total de 132 g/m². On a mesuré la vitesse de transpercement et la résistance au remouillage. Les résultats sont donnés dans le tableau 1.

### Exemple 1

On a ajouté au voile de surface de la couche-culotte de l'exemple comparatif A un voile de surface en un matériau composite selon l'invention comprenant deux couches de fibres de polyester liées ensemble par aiguilletage. Les fibres de polyester de la couche interne étaient de 6,6 deniers et cette couche avait un grammage de 41 g/m². Les fibres de polyester de la couche externe étaient de 3,3 deniers et cette couche avait un grammage de 22 g/m². Cette couche externe est disposée en contact avec le voile de surface classique de la couche-culotte.

On a déterminé la vitesse de transpercement et la résistance au remouillage. Les résultats sont donnés dans le tableau 1.

### Exemple comparatif 1a

On a réalisé la même structure que dans l'exemple 1, mais en inversant la position des couches du matériau composite selon l'invention par rapport au voile de surface classique de la couche-culotte, c'est-à-dire en plaçant directement la couche en fibres de 6,6 deniers sur le voile de surface classique de la couche-culotte. On a déterminé la vitesse de transpercement et la résistance au remouillage. Les résultats sont donnés dans le tableau 1.

### Exemple 2

On a réalisé la même structure que dans l'exemple 1 mais en utilisant pour la couche externe en fibres de plus faible dernier des fibres de polyester de 1,5 deniers. Cette couche externe avait un grammage de 22 g/m². Cette couche externe en fibres de 1,5 deniers était disposée directement sur le voile de surface classique de la couche-culotte. On a déterminé la vitesse de transpercement et la résistance au remouillage. Les résultats sont donnés dans le tableau 1.

### Exemple comparatif 2a

On a réalisé la même structure que dans l'exemple 2, mais en inversant la position des couches du matériau composite selon l'invention par rapport au voile de surface classique de la couche-culotte, c'est-à-dire en plaçant directement la couche de fibres de 6,6 deniers sur le voile de surface classique de la couche-culotte. On a déterminé la vitesse de transpercement et la résistance au remouillage. Les résultats sont donnés dans le tableau 1.

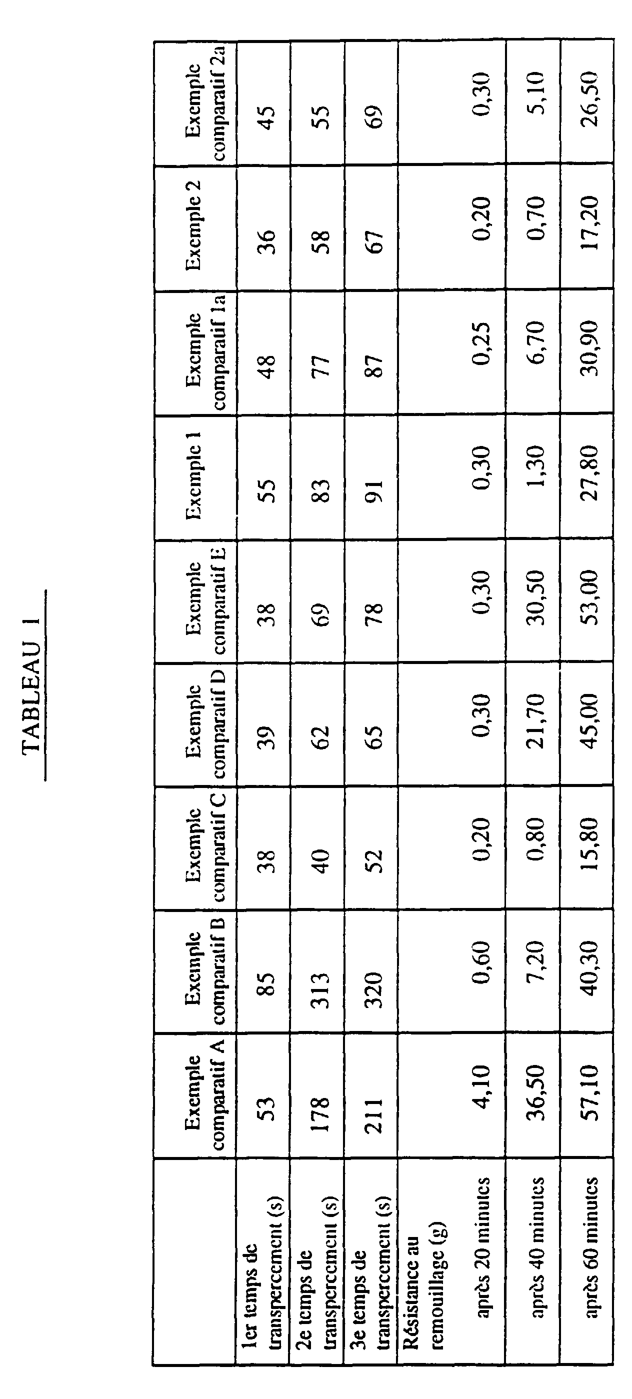

Les temps de transpercement et la résistance au remouillage ont été déterminés de la façon suivante :

Les produits finis sont conditionnés à 23°C et 50% d'humidité relative pendant 24 heures avant les essais.

On place au centre du voile de surface de l'article testé une plaque en plexiglas® de 7x7 cm perforée en son centre. On verse dans l'orifice de la plaque 100cm³ d'une solution saline à 9g/l de chlorure de sodium dans de l'eau distillée au moyen d'une ampoule à décanter en réglant l'écoulement de l'ampoule pour avoir un niveau constant haut dans l'orifice de la plaque. On mesure le temps écoulé entre le début de l'introduction de la solution saline et l'instant auquel la solution saline a disparu dans l'article. Le temps mesuré constitue le premier temps de transpercement.

On pèse alors six papiers filtres DIMAR ED 939® découpés en carrés de 10,2 x 10,2 cm. On place ensuite, après avoir ôté la plaque perforée, sur le voile de surface de l'article testé, un poids de 3,5 kg de 10,2 x 10,2 cm pendant 10 minutes. Une fois les 10 minutes écoulées, on place sous le poids les six papiers filtres et on laisse encore pendant 10 minutes. A la fin de cette période on retire le poids et les papiers filtres. On pèse les papiers filtres. La différence de poids, en grammes, entre la première et la seconde pesée donne un mesure de la résistance au remouillage après 20 minutes.

On recommence deux fois la procédure ci-dessus avec le même article, en utilisant respectivement 24 filtres après la deuxième addition de solution saline et 30 filtres après la troisième addition. On obtient ainsi les deuxième et troisième temps de transpercement ainsi que la résistance au remouillage après 40 et 60 minutes.

Une comparaison des résultats obtenus entre les exemples 1 et 2 d'une part et l'exemple comparatif A d'autre part, montre que l'utilisation d'un non-tissé composite selon l'invention en complément d'un voile de surface classique, améliore très nettement la vitesse de transpercement du liquide (plus de 50% au 3^{e} essai), bien que le grammage total du voile de surface soit augmenté de 63 g/m²;

en outre la comparaison des résultats obtenus entre les exemples 1 et 2 et les exemples comparatifs 1a et 2a montre que plus on augmente l'écart dans la décroissance entre les valeurs des deniers de la couche interne et de la couche externe superposées, meilleures sont à la fois la vitesse de transpercement et la résistance au remouillage. On observe en outre dans les exemple comparatifs 1a et 2a que lorsque la position respective des deux couches est inversée, les résistances au remouillage s'en trouvent réduites. Enfin la comparaison des résultats obtenus aux exemples comparatifs C, D et E avec ceux de l'exemple comparatif B montre que l'utilisation en remplacement d'un voile de surface classique d'un voile composite ayant un grammage sensiblement constant, formé de plusieurs couches de matériau non-tissé de même denier, permet d'améliorer la vitesse de transpercement, la résistance au remouillage ne se trouve particulièrement renforcée que lorsque le denier du voile composite est élevé, donc lorsque le gradient entre la porosité moyenne du coussin absorbant et du voile de surface est le plus grand, et pour un grammage total important.

## Revendications

1. Procédé pour améliorer la vitesse de transpercement par les fluides corporels et la résistance au remouillage d'un article d'hygiène absorbant comprenant une couche externe (11) imperméable aux fluides corporels, un coussin absorbant (12), de surface (13) et éventuellement une bande de zone d'entrejambe (15) perméables aux fluides corporels, caractérisé en ce que ledit voile de surface (13) ou ladite bande de zone d'entrejambes (15) est constitué(e) d'un matériau non-tissé multicouche comprenant au moins deux couches superposées (1-5) de fibres textiles naturelles ou synthétiques, réunies entre elles, les fibres des couches successives ayant un denier qui décroît progressivement, dans un sens donné, d'une couche successive à l'autre, et la couche de fibres de plus faible denier étant destinée à former la couche externe du voile ou de la bande de zone d'entrejambes la plus proche du coussin absorbant.

2. Procédé selon la revendication 1 dans lequelle les fibres des couches successives sont de même nature ou de nature différente.

3. Procédé selon la revendication 1 ou 2 dans lequelle le matériau comprend des couches non traitées et des couches traitées avec un agent de surface ou d'ensimage hydrophilique.

4. Procédé selon la revendication 3, dans lequelle les couches du matériau sont alternativement des couches traitées et non traitées.

5. Procédé selon l'une quelconque des revendications précédentes dans lequelle les fibres des couches sont choisies parmi les fibres de cellulose, viscose, polyéthylène, polypropylène, polyester et de copolymères d'éthylène-propylène.

6. Procédé selon l'une quelconque des revendications précédentes dans lequelle le matériau comprend deux couches.

7. Procédé selon la revendication 6 dans laquelle les fibres de la couche de fibres de plus grand denier ont un denier compris entre 3,3 et 6,6, et les fibres de la couche de fibres de plus faible denier ont un denier compris entre 1,5 et 3,3.

8. Procédé selon l'une quelconque des revendications précédentes dans lequelle les couches sont réunies par aiguilletage ou enchevêtrement par jets d'eau.

9. Article d'hygiène absorbant comprenant une couche externe (11) imperméable aux fluides corporels, un coussin absorbant (12) perméable aux fluides corporels et lié à la couche externe, et un voile de surface (13) perméable aux fluides corporels et lié à la couche externe, caractérisé en ce que le voile (13) est constitué par un matériau non-tissé multicouche comprenant au moins deux couches superposées (1-5) de fibres textiles naturelles ou synthétiques, réunies entre elles, les fibres des couches successives ayant un denier qui décroît progressivement, dans un sens donné, d'une couche successive à l'autre et la couche (5) ayant les fibres de plus faible denier étant située directement sur la surface supérieure ou interne du coussin absorbant (12).

10. Article d'hygiène absorbant comprenant une couche externe (11) imperméable aux fluides corporels, un coussin absorbant (12) perméable aux fluides corporels et lié à la couche externe, un voile de surface (13) perméable aux fluides corporels et lié à la couche externe et une bande de zone d'entre-jambes (15) perméable aux fluides corporels, de largeur analogue à la largeur du coussin absorbant (12) dans sa zone d'entre-jambes et de même longueur que ce coussin absorbant, caractérisé en ce que la bande de zone d'entre-jambe (15) est constituée d'un matériau non-tissé multicouche comprenant au moins deux couches superposées de fibres textiles naturelles ou synthétiques. réunies entre elles, les fibres des couches successives ayant un denier qui décroît progressivement, dans un sens donné, d'une couche successive à l'autre, la couche de plus grand denier constituant la couche interne la plus éloignée du coussin absorbant (12) et la couche de plus faible denier constituant la couche externe la plus proche du coussin absorbant.

11. Article d'hygiène absorbant selon la revendication 10 caractérisé en ce que la bande de zone d'entre-jambes (15) est située sur la surface interne du voile de surface (13).

12. Article d'hygiène absorbant selon la revendication 10 caractérisé en ce que la bande de zone d'entre-jambes (15) est située entre le coussin absorbant (12) et le voile de surface (13).

13. Article d'hygiène absorbant selon la revendication 11 caractérisé en ce que la bande de zone d'entre-jambes (15) est liée au voile de surface (13).

14. Article d'hygiène absorbant selon la revendication 12 caractérisé en ce que la bande de zone d'entre-jambes (15) est liée soit au voile de surface soit au coussin absorbant.

15. Article d'hygiène absorbant selon la revendication 13 ou 14 caractérisé en ce que la bande de zone d'entre-jambes est liée par collage, thermoscellage, scellage aux ultrasons ou aiguilletage.

16. Article selon l'une quelconque des revendications 9 à 15 caractérisé en ce que les fibres des couches successives sont de même nature ou de nature différente.

17. Article selon l'une quelconque des revendications 9 à 16 caractérisé en ce que le matériau non-tissé multicouche comprend des couches non traitées et des couches traitées avec un agent de surface ou d'ensimage hydrophilique.

18. Article selon la revendication 17 caractérisé en ce que les couches du matériau sont alternativement des couches traitées et non traitées.

19. Article selon l'une quelconque des revendications 9 à 18 caractérisé en ce que les fibres des couches sont choisies parmi les fibres de cellulose, viscose, polyéthylène, polypropylène, polyester et de copolymères d'éthylène-propylène.

20. Article selon l'une quelconque des revendications 9 à 19 caractérisé en ce que le matériau comprend deux couches.

21. Article selon la revendication 20 caractérisé en ce que les fibres de la couche de fibres de plus grand denier ont un denier compris entre 3,3 et 6,6, et les fibres de la couche de fibres de plus faible denier ont un denier compris entre 1,5 et 3,3.

22. Article selon l'une quelconque des revendications 9 à 21 caractérisé en ce que les couches sont réunies par aiguilletage ou enchevêtrement par jets d'eau.

## Patentansprüche

1. Verfahren zum Verbessern der
Durchdringungsgeschwindigkeit der Körperflüssigkeiten durch einen absorbierenden Hygieneartikels sowie des Widerstandes gegen ein erneutes Naßwerden desselben, der eine für die Körperflüssigkeiten undurchdringliche äußere Lage (11), ein absorbierendes Kissen (12), einen Oberflächenflor (13) und ggf. ein für die Körperflüssigkeiten durchlässiges Schrittzonenband (15) aufweist,
dadurch **gekennzeichnet**, daß der Oberflächenflor (13) oder das Schrittzonenband (15) aus einem mehrschichtigen, nichtgewebten Material gebildet ist, welches mindestens zwei übereinander angebrachte Lagen (1-9) von natürlichen oder synthetischen Textilfasern umfaßt, die miteinander verbunden sind, wobei die Fasern der aufeinanderfolgenden Lagen einen Denier besitzen, der progressiv in einer gegebenen Richtung von einer Folgeschicht zur anderen abnimmt, und wobei die Lage mit Fasern mit dem geringsten Denier dazu bestimmt ist, diejenige äußere Lage des Flors oder des Schrittzonenbandes zu bilden, die dem absorbierenden Kissen am nächsten liegt.

2. Verfahren nach Anspruch 1, bei dem die Fasern der aufeinanderfolgenden Lagen von der gleichen Art oder einer unterschiedlichen Art sind.

3. Verfahren nach Anspruch 1 oder 2, bei dem das Material nichtbehandelte Lagen und Lagen umfaßt, die mit einem Oberflächenagens oder einem hydrophilen Schmälzagens behandelt sind.

4. Verfahren nach Anspruch 3, bei dem die Lagen des Materials alternierend behandelte und nichtbehandelte Lagen sind.

5. Verfahren nach einem beliebigen der vorhergehenden Ansprüche, bei dem die Fasern der Lagen unter den Fasern aus Zellulose, Viskose, Polyäthylen, Polypropylen, Polyester und den Copolymeren von Äthylen-Propylen gewählt werden.

6. Verfahren nach einem beliebigen der vorhergehenden Ansprüche, bei dem das Material zwei Lagen umfaßt.

7. Verfahren nach Anspruch 6, bei dem die Fasern der Faserlagen mit dem größeren Denier einen Denier zwischen 3,3 und 6,6 aufweisen, und die Fasern der Faserlagen mit dem geringeren Denier einen Denier zwischen 1,5 und 3,3 aufweisen.

8. Verfahren nach einem beliebigen der vorhergehenden Ansprüche, bei dem die Lagen durch Nadelung oder Verblockung mit Wasserstrahlen verbunden werden.

9. Absorbierender Hygieneartikel, der eine für die Körperflüssigkeiten undurchlässige äußere Lage (11), ein absorbierendes Kissen (12), das für die Körperflüssigkeiten durchlässig und mit der äußeren Lage verbunden ist, und einen Oberflächenflor (13) aufweist, der für die Körperflüssigkeiten durchlässig und mit der äußeren Lage verbunden ist,
dadurch **gekennzeichnet,** daß der Flor (13) aus einem mehrschichtigen, nichtgewebten Material besteht, welches mindestens zwei übereinander angebrachte Lagen (1-5) natürlicher oder synthetischer Textilfasern umfaßt, die miteinander verbunden sind, wobei die aufeinanderfolgenden Faserlagen einen Denier aufweisen, der progressiv in eine gegebene Richtung von einer Folgelage zur anderen abnimmt, und wobei die Lage (5) mit den Fasern des geringsten Deniers direkt auf der oberen bzw. inneren Oberfläche des absorbierenden Kissens (12) angebracht ist.

10. Absorbierender Hygieneartikel, der eine für die Körperflüssigkeiten undurchlässige äußere Lage (11), ein absorbierendes Kissen (12), das für die Körperflüssigkeiten durchlässig und mit der äußeren Lage verbunden ist, einen Oberflächenflor (13), der für die Körperflüssigkeiten durchlässig und mit der äußeren Lage verbunden ist, und ein Schrittzonenband (15) aufweist, das für die Körperflüssigkeiten durchlässig ist, mit einer Breite, die der Breite des absorbierenden Kissens (12) in der Schrittzone analog ist, und mit gleicher Länge wie das absorbierende Kissen,
dadurch **gekennzeichnet**, daß das Schrittzonenband (15) aus einem mehrschichtigen, nichtgewebten Material besteht, welches mindestens zwei übereinander angebrachte Lagen aus natürlichen oder synthetischen Textilfasern aufweist, die miteinander verbunden sind, wobei die aufeinanderfolgenden Lagen einen Denier aufweisen, der progressiv in einer gegebenen Richtung von einer Folgelage zur anderen abnimmt, wobei die Lage mit dem größeren Denier die innere, am weitesten vom absorbierenden Kissen (12) befindliche Lage ist, und die Lage mit dem geringsten Denier die äußere, dem absorbierenden Kissen am nächsten kommende Lage ist.

11. Absorbierender Hygieneartikel nach Anspruch 10,
dadurch **gekennzeichnet**, daß das Schrittzonenband (15) auf der inneren Oberfläche des Oberflächenflors (13) plaziert ist.

12. Absorbierender Hygieneartikel nach Anspruch 10,
dadurch **gekennzeichnet**, daß das Schrittzonenband (15) zwischen dem absorbierenden Kissen (12) und dem Oberflächenflor (13) plaziert ist.

13. Absorbierender Hygieneartikel nach Anspruch 11,
dadurch **gekennzeichnet**, daß das Schrittzonenband (15) mit dem Oberflächenflor verbunden ist.

14. Absorbierender Hygieneartikel nach Anspruch 12,
dadurch **gekennzeichnet**, daß das Schrittzonenband (15) entweder mit dem Oberflächenflor oder dem absorbierenden Kissen verbunden ist.

15. Absorbierender Hygieneartikel nach Anspruch 13 oder 14,
dadurch **gekennzeichnet**, daß das Schrittzonenband durch Klebung, Wärmeversiegelung, Versiegelung durch Ultraschall oder Nadelung verbunden ist.

16. Artikel gemäß irgendeinem der Ansprüche 9 bis 15,
dadurch **gekennzeichnet**, daß die Fasern der aufeinanderfolgenden Lagen gleicher oder unterschiedlicher Natur sind.

17. Artikel gemäß irgendeinem der Ansprüche 9 bis 16,
dadurch **gekennzeichnet**, daß das mehrschichtige, nichtgewebte Material mit einem Oberflächenagens oder einer hydrophilen Schmälzmasse behandelte oder nichtbehandelte Lagen aufweist.

18. Artikel nach Anspruch 17,
dadurch **gekennzeichnet**, daß die Lagen des Materials alternierend behandelte Schichten und nichtbehandelte Schichten sind.

19. Artikel gemäß irgendeinem der Ansprüche 9 bis 18,
dadurch **gekennzeichnet**, daß die Fasern der Lagen unter den Fasern als Zellulose, Viskose, Polyäthylen, Polypropylen, Polyester und aus Copolymeren von Äthylen-Propylen gewählt sind.

20. Artikel gemäß irgendeinem der Ansprüche 9 bis 19,
dadurch **gekennzeichnet**, daß das Material zwei Lagen umfaßt.

21. Artikel nach Anspruch 20,
dadurch **gekennzeichnet**, daß die Fasern der Faserlage mit dem größeren Denier einen Denier zwischen 3,3 und 6,6 aufweisen, während die Fasern der Faserlage mit dem geringeren Denier einen Denier zwischen 1,5 und 3,3 aufweisen.

22. Artikel gemäß irgendeinem der Ansprüche 9 bis 21,
dadurch **gekennzeichnet**, daß die Lagen durch Nadelung oder Verblockung mit Wasserstrahlen verbunden sind.

## Claims

1. Process for improving break-through by the body fluids and reduction of return of the body fluids of an absorbent article of hygiene comprising an outer layer (11) which is impervious to body fluids, an absorbent pad (12), a surface web (13) and eventually a crotch region strip (15) permeable to body fluids , characterized in that the said surface web (13) or the said crotch region strip (15) is constituted of a multilayer nonwoven material comprising at least two superposed layers (1-9) of natural or synthetic textile fibers, joined together , in which the fibers of the successive layers have a denier which decreases progressively, in a given direction, from one successive layer to the other , and the fiber layer of lower denier being intended to form the outer layer of the surface web or crotch region strip closest to the absorbent pad.

2. Process according to claim 1, wherein the fibers of the sucessive layers are of the same kind or of different kinds.

3. Process according to claim 1 or 2, wherein the multilayer nonwoven material comprises untreated layers and layers treated with a hydrophilic surface or oiling agent.

4. Process according to claim 3, wherein the layers are treated and untreated layers alternately.

5. Process according to anyone of the preceding claims, wherein the fibers of the layers are chosen from cellulose, viscose, polyethylene, polypropylene, polyester and ethylene-propylene copolymer fibers.

6. Process according to anyone of the preceding claims, wherein the multilayer nonwoven material comprises two layers.

7. Process according to claim 6, wherein the fibers of the layer of fibers of higher denier have a denier of between 3.3 and 6.6 and the fibers of the layer of fibers of lower denier have a denier of between 1.5 and 3.3.

8. Process according to anyone of the preceding claims , wherein the layers are joined by needling or tangling by jets of water.

9. Absorbent article of hygiene comprising an outer layer (11) which is impervious to body fluids, an absorbent pad (12) which is permeable to body fluids and bonded to the outer layer, and a surface web (13) which is permeable to body fluids and bonded to the outer layer,characterized in that the web (13) is constituted by a multilayer nonwoven material comprising at least two superposed layers of natural or synthetic textile fibers, joined together, in which the fibers of the successive layers having a denier which decreases progressively, in a given direction, from one successive layer to the other, and the layer (5) having the fibers of lower denier being situated directly on the upper or internal surface of the absorbent pad (12).

10. Absorbent article of hygiene comprising an outer layer (11) impermeable to body fluids, an absorbent pad (12) permeable to body fluids and bonded to the outer layer,a surface web (13) permeable to body fluids and bonded to the outer layer and a crotch region strip (15) permeable to body fluids, with a width similar to the width of the absorbent pad (12) in its crotch region and of the same length as this absorbent pad, charaterized in that the crotch region strip (15) consists of a multilayer nonwoven, material comprising at least two superposed layers of natural or synthetic textile fibers, joined together, in which the fibers of the successive layers have a denier which decreases progressively, in a given direction, from one successive layer to the other, the layer of higher denier forming the inner layer furthest away from the aborbent pad (12) and the layer of lower denier forming the outer layer closest the the absorbent pad.

11. Absorbent article of hygiene as claimed in claim 10, characterized in that the crotch region strip (15) is situated on the inner surface of the surface web (13).

12. Absorbent article of hygiene as claimed in claim 10, characterized in that the crotch region strip (15) is situated between the absorbent pad (12) and the surface web (13).

13. Absorbent article of hygiene as claimed in claim 11, characterized in that the crotch region (15) is bonded to the surface web (13).

14. Absorbent article of hygiene as claimed in claim 12, charcaterized in that the crotch region (15) is bonded either to the surface web or to the absorbent pad.

15. Absorbent article of hygiene as claimed in claim 13 or 14, characterized in that the crotch region strip is bonded by adhesive bonding, heat-sealing, ultrasonic welding or needling.

16. Absorbent article of hygiene as claimed in anyone of claims 9 to 15, characterized in that the fibers of the sucessive layers are of the same kind or of different kinds.

17. Absorbent article of hygiene as claimed in anyone of claims 1 to 16, characterized in that the multilayer nonwoven material comprises untreated layers and layers treated with a hydrophilic surface or oiling agent.

18. Absorbent article of hygiene as claimed in anyone of claims 9 to 18, characterized in that the layers are treated and untreated layers alternately.

19. Absorbent article of hygiene as claimed in anyone of claims 9 to 18, characterized in that the fibers of the layers are chosen from cellulose,viscose, polyethylene, polypropylene, polyester and ethylene-propylene copolymer fibers.

20. Absorbent article of hygiene as claimed in anyone of claims 9 to 19, characterized in that the multilayer nonwoven material comprises two layers.

21. Absorbent article of hygiene as claimed in claim 20, characterized in that the fibers of the layer of fibers of higher denier have a denier of between 3.3 and 6.6 and the fibers of the layer of fibers of lower denier have a denier of between 1.5 and 3.3.

22. Absorbent article of hygiene as claimed in anyone of claims 9 to 21 , characterized in that the layers are joined by needling or tangling by jets of water.
